# EUROPEAN PATENT APPLICATION

(11) **EP 2 275 090 A2**
(43) Date of publication of application: **19.01.2011**
(21) Application number: 10177159.0
(22) Date of filing: 05.12.2003
(51) Int. Cl.: A61K 9/16

(54) **Microparticles prepared using an ionic liquid**

(30) Priority: 06.12.2002 GB 0228571
(62) Divisional of application: 03782314.3
(71) Applicant: Novartis AG, 4056 Basel (CH); Novartis Pharma GmbH, 1230 Wien (AT)
(72) Inventor: Ausborn, Michael, 79540, Lörrach (DE); Kissel, Thomas, 79219, Staufen (DE); Küsters, Ernst, 79427, Eschbach (DE)
(74) Representative: Lehmeier, Thomas Jürgen

(57) **Abstract**

Microparticles comprising at least one active agent embedded within a biocompatible, biodegradable polymeric matrix, wherein said microparticles are prepared with an ionic liquid.

## Description

### Field of the Invention

The present invention provides microparticles comprising at least one active agent embedded within a biocompatible, biodegradable polymeric matrix, wherein said microparticles are prepared with an ionic liquid.

### Background of the Invention

It is advantageous to prepare microparticles having a biologically active or pharmaceutically active agent within a biocompatible, biodegradable polymer to provide sustained or delayed release of the active agent. Typically, the microparticles are prepared by dissolving, dispersing or emulsifying an active agent in a solvent containing a polymer. The solvent is then removed from the microparticles.

U.S. Patent No. 6,228,398 describes microparticles containing a bioactive agent in a polymer matrix. The microparticles are prepared using a solvent which exhibits a high vapor pressure to ensure that the final solvent extraction step can be carried out quickly. Suitable solvents are listed as ethyl acetate, dichloromethane, chloroform and propylene carbonate.

Bodmeier et al., Int. J. Pharmaceutics, Vol. 43, pp. 179-186 (1988), describes the preparation of microparticles containing quinidine or quinidine sulfate as the active agent and poly(D,L-lactide) as the binder using a variety of solvents including methylene chloride, chloroform and benzene as well as mixtures of methylene chloride and a water miscible liquid, such as acetone, ethyl acetate, methanol, dimethylsulfoxide, chloroform or benzene to enhance drug content.

Beck et al., Biol. Reprod., Vol. 28, pp. 186-195 (1983), describes a process for encapsulating norethisterone in a copolymer of D,L-lactide and glycolide by dissolving both the co-polymer and the norethisterone in a mixture of chloroform and acetone that is added to a stirred cold aqueous solution of polyvinyl alcohol to form an emulsion and the volatile solvents removed under reduced pressure to yield microcapsules.

All of the microencapsulation processes described above employ volatile solvents. In many instances, the solvents are halogenated hydrocarbons, such as chloroform and ethylene chloride, which are especially undesirable because of their general toxicity, possible carcinogenic activity and environmental concern.

We have now found after exhaustive testing that it is possible to make microparticles without using volatile solvents.

### Summary of the Invention

The invention provides microparticles comprising at least one active agent embedded within a biocompatible, biodegradable polymeric matrix, wherein said microparticles are prepared with an ionic liquid.

According to another aspect the invention provides a method for preparing microparticles comprising dissolving, dispersing or emulsifying an active agent in a biocompatible, biodegradable polymer and an ionic liquid, to form a mixture; and removing the ionic liquid from the mixture, thereby forming microparticles containing the active agent embedded within a polymeric matrix.

Microparticles prepared according to the invention have the following advantages:
(i) an active agent or drug substance and a polymer which are used to prepare the microparticles may co-dissolve in the ionic liquid which may eliminate an emulsification or drug suspending step in a process to prepare the microparticles;
(ii) the distribution of an active agent or drug substance in a polymer matrix may be more homogeneous in the microparticles;
(iii) the microparticles are free from halogenated hydrocarbon residues;
(iv) the microparticles are prepared essentially free of volatile solvents since the ionic liquids have essentially no vapor pressure;
(v) the particle size, shape, and density of the microparticles may be controlled by the choice of ionic liquid;
(vi) an active agent and a polymer may be co-dissolved in the ionic liquid; and
(vii) a peptide or protein co-dissolved with a polymer in the ionic liquid may not undergo potentially devastating stress conditions during emulsification or suspension of a protein/water phase in a polymer/ionic liquid phase.

### Description of the Invention

The microparticles of the invention comprising at least one active agent embedded within a biocompatible, biodegradable polymeric matrix are prepared with at least one ionic liquid. As used herein, the terms "microparticles" and "microspheres" include solid particles that contain an active agent dispersed or dissolved within a biodegradable, biocompatible polymer that serves as the matrix of the particle. As used herein, the term "biodegradable" means a material that should degrade by bodily processes to products readily disposable by the body and should not accumulate in the body. The products of the biodegration should also be biocompatible with the body. As used herein, the term "biocompatible" means not toxic to the human body and is pharmaceutically acceptable.

The biodegradable, biocompatible polymer may be a synthetic polymer or natural polymer. As used herein, "polymer" means at least one monomer is used in the polymerization, thus, "polymer" includes co-polymers, terpolymers, tetrapolymers, etc. The polymer may be cross-linked or non-cross-linked. Preferably, the degree of cross-linking is less than 5%, more preferably, less than 1%. Suitable polymers include the following:
a) linear or branched polyesters which are linear chains radiating from a polyol moiety, e.g., glucose;
b) polyesters, such as D-, L- or racemic polylactic acid, polyglycolic acid, polyhydroxybutyric acid, polycaprolactone, polyalkylene oxalate, polyalkylene glycol esters of acids of the Kreb's cycle, e.g., citric acid cycle, and the like and combinations thereof;
c) polymers of organic ethers, anhydrides, amides and orthoesters
d) co-polymers of organic esters, ethers, anhydrides, amides, and orthoesters by themselves or in combination with other monomers; and
e) polyvinylalcohol.

Preferred examples of polymers which form the polymeric matrix include poly(glycolic acid), poly(D,L-lactic acid), poly(L-lactic acid), co-polymers of the foregoing, poly(DL-lactide-coglycolide)-glucose (PLG-Glu) and the like. Various commercially available poly(lactide-coglycolide) materials (PLGA) may be used in the method of the present invention. For example, poly(D,L-lactic-co-glycolic acid) is commercially available from Medisorb Technologies International L.P. A suitable product commercially available from Medisorb is a 50:50 poly (D,L-lactic co-glycolic acid) known as MEDISORB.RTM. 50:50 DL. This product has a mole percent composition of 50% lactide and 50% glycolide. Other suitable commercially available products are MEDISORB.RTM. 65:35 DL, 75:25 DL, 85:15 DL and poly(D,L-lactic acid) (D,1-PLA). Poly(D,L lactic acids), e.g. R202 which has a number average molecular weight of approximately 16000, and poly(lactide-co-glycolides) are commercially available from Boehringer Ingelheim under its Resomer mark, e.g., PLGA 50:50, e.g. Resomer RG 502 which has a number average molecular weight of approximately 12000, PLGA 75:25, e.g. Resomer RG 752 which has a number average molecular weight of approximately 16000, D,1-PLA, e.g. Resomer RG 206, and poly (DL-lactide-co-glycolide), e.g. RG 505 which has a number average molecular weight of approximately 80000. PLG-GLU may have a number average molecular weight of approximately 55200. Other suitable commercially available products are from Birmingham Polymers. These co-polymers are available in a wide range of molecular weights and ratios of lactic acid to glycolic acid.

The most preferred polymer for use in preparing the microparticles of the invention is the copolymer poly(D,L-lactide-co-glycolide). It is preferred that the molar ratio of lactide to glycolide in such a co-polymer be in the range of from about 85:15 to about 50:50.

The molecular weight of the polymer should be high enough to form a film, preferably from about 5,000 daltons to about 500,000 daltons. However, since the properties of the film are also partially dependent on the particular polymer being used, it is difficult to specify an appropriate molecular weight range for all polymers. The molecular weight of a polymer may also effect the biodegradation rate of the polymer. By an appropriate selection of polymer and other variables, such as process conditions, excipients, loading of active agent, and water content, microparticles can be made which exhibit diffusional release and/or biodegradation release properties.

The active agent is dispersed or dissolved in the polymeric matrix of the microparticles. Any active agent may be used. Example of active agents include, but are not limited to, peptides or proteins, hormones, analgesics, anti-migraine agents, anti-coagulant agents, narcotic antagonists, chelating agents, anti-anginal agents, chemotherapy agents, sedatives, anti-neoplastics, prostaglandins and anti-diuretic agents, drug compounds acting on the central nervous system, such as cerebral stimulants, e.g., methylphenidate; pain management active agents; alkaloids, such as opiates, e.g., morphine; cardiovascular drugs, such as nitrates; and agents for treating rheumatic conditions. It is further appreciated that the active agents include, but are not limited to, proteins or peptides, such as insulin, calcitonin, calcitonin gene-regulating protein, parathyroid hormone, GLP-1, atrial natriuretic protein, colony-stimulating factor, GM-CSF, betaseron, erythropoietin, interferons such as alpha- ,beta- or gamma-interferon, human growth hormone, octreotide, somatropin, somatotropin, somastostatin, insulin-like growth factor (somatomedins), luteinizing hormone releasing hormone, tissue plasminogen activator, growth hormone releasing hormone, oxytocin, estradiol, growth hormones, leuprolide acetate, factor VIII, interleukins, such as interleukin-2, 3, 6, and 14, and analogues and antagonists thereof; analgesics, such as fentanyl, sufentanil, butorphanol, buprenorphine, levorphanol, morphine, hydromorphone, hydrocodone, oxymorphone, methadone, lidocaine, bupivacaine, diclofenac, naproxen, paverin and analogues thereof; anti-migraine agents, such as sumatriptan, ergot alkaloids and analogues thereof; anti-coagulant agents, such as heparin, hirudin and analogues thereof; anti-emetic agents, such as scopolamine, ondansetron, domperidone, metoclopramide and analogues thereof; cardiovascular agents, anti-hypertensive agents and vasodilators, such as diltiazem, clonidine, nifedipine, verapamil, isosorbide-5-mononitrate, organic nitrates, agents used in treatment of heart disorders and analogues thereof; sedatives, such as benzodiazepines, phenothiozines and analogues thereof; chelating agents; anti-diuretic agents, such as desmopressin, vasopressin and analogues thereof; anti-anginal agents, such as nitroglycerine and analogues thereof; anti-neoplastics, such as fluorouracil, bleomycin and analogues thereof; prostaglandins and analogues thereof; and chemotherapy agents, such as vincristine and analogues thereof. The microparticles may contain a combination of active agents. For example, microparticles which contain at least two active agents may be desirable for combination therapies.

The loading of microparticles with active agent may be detected by UV/Vis spectroscopy and/or gel electrophoresis (native PAGE) and/or or High Pressure Liquid Chromatography (HPLC).

The amount of active agent depends on the type of active agent and the condition to be treated. Preferably, the active agent is present in an amount of from about 0.1 to about 90 weight percent, based on the total weight of the polymeric matrix. More preferably, the active agent is present in an amount of from about 2 to about 75 weight percent. Most preferably, in the case where the active agent is a protein, peptide, or hormone, the amount of active agent in the microparticles is from about 1 to about 30 weight percent, preferably 1-20 weight percent, more preferably 1-10 weight percent, and most preferably about 5 weight percent, based on the total weight of the polymeric matrix.

In addition to the active agent, the microparticles may contain an enhancer compound or a sensitiser compound, in order to modify the bioavailability or therapeutic effect of the active agent. As used herein, "enhancer" refers to a compound which is capable of enhancing the absorption and/or bioavailability of an active agent. Enhancers include, but are not limited to, medium chain fatty acids; salts, esters, ethers and derivatives thereof, including glycerides and triglycerides; non-ionic surfactants such as those that can be prepared by reacting ethylene oxide with a fatty acid, a fatty alcohol, an alkylphenol or a sorbitan or glycerol fatty acid ester; cytochrome P450 inhibitors, P-glycoprotein inhibitors and the like; and mixtures of two or more enhancers.

Ionic liquids are characterized by a positively charged cation and a negatively charged anion. Generally, any molten salt or mixture of molten salts is considered an ionic liquid. Ionic liquids typically have essentially no vapor pressure, good heat transfer characteristics, are stable over a wide temperature range, and are capable of dissolving a wide range of material in high concentrations. As used herein, "essentially no vapor pressure" means that the ionic liquid exhibits a vapor pressure of less than about 1 mm/Hg at 25 °C, preferably less than about 0.1 mm/Hg at 25 °C.

With respect to the type of ionic liquid, a wide variety of possibilities exist. However, the preferred ionic liquids are liquid at relatively low temperatures, for example, below the melting point of the compound. Preferably, the ionic liquid has a melting point of less than 250 °C, more preferably less than 100 °C. Most preferably, the ionic liquid has a melting point of less than 30 °C and is a liquid at room temperature. It is within the scope of the invention to prepare an ionic liquid having any number of desirable properties, e.g. an ionic liquid that dissolves a specific polymer which is used to prepare microparticles.

With regard to viscosity of the ionic liquid, it is important that the viscosity of the ionic liquid not be too high to prevent a homogeneous solution or dispersion of the active agent in the ionic liquid. Preferably, the ionic liquid has a viscosity of less than 500 centipoise (cP), more preferably, less than 300 cP, and most preferably less than 100 cP, as determined at 25 °C.

The cation present in the ionic liquid can be a single species or a plurality of different species. Both of these embodiments are intended to be embraced, unless otherwise specified, by the use of the singular expression "cation". The cations of the ionic liquid include organic and inorganic cations. Examples of cations include quaternary nitrogen-containing cations, phosphonium cations and sulfonium cations.

The quaternary nitrogen-containing cations are not particularly limited and embrace cyclic, aliphatic, and aromatic quaternary nitrogen-containing cations. Preferably, the quaternary nitrogen-containing cation is an n-alkyl pyridinium, a dialkyl imidazolium or an alkylammonium of the formula R'₄₋ₓ NHₓ, wherein X is 0-3 and each R' is independently an alkyl group having 1-18 carbon atoms. It is believed that unsymmetrical cations can provide for lower melting temperatures. The phosphonium cations are not particularly limited and embrace cyclic, aliphatic and aromatic phosphonium cations. Preferably, the phosphonium cations include those of the formula R"₄₋ₓ PHₓ, wherein X is 0-3, and each R" is an alkyl or aryl group, such as an alkyl group having 1-18 carbon atoms or a phenyl group. The sulfonium cations are not particularly limited and embrace cyclic, aliphatic and aromatic sulfonium cations. Preferably, the sulfonium cations include those of the formula R"'_{3-X} SH_{X}, wherein X is 0-2 and each R"' is an alkyl or aryl group, such as an alkyl group having 1-18 carbon atoms or a phenyl group. Preferred cations include 1-hexyl(pyridinium, ammonium, imidazolium, 1-ethyl-3-methylimidazolium, 1-butyl-3-methylimidazolium, phosphonium and *N-*butylpyridinium.

The anion used in the ionic liquid is not particularly limited and includes organic and inorganic anions. Generally the anion is derived from an acid, especially a Lewis acid. The anions are typically metal halides as described in more detail below, boron or phosphorus fluorides, alkylsulfonates including fluorinated alkyl sulfonates, such as nonafluorobutanesulfonate; and carboxylic acid anions, such as trifluoroacetate and heptafluorobutanoate. The anion is preferably Cl⁻, Br⁻, NO₂⁻, NO₃⁻, AlCl₄⁻, BF₄⁻, PF₆⁻, CF₃COO⁻ , CF₃SO₃⁻, (CF₃SO₂)₂N⁻, OAc⁻, CuCl₃⁻, GaBr₄⁻, GaCl₄⁻, SbF₆⁻ and CH₃SO₄.

Examples of ionic liquids include, but are not limited to, imidazolium salts, pyridium salts, ammonium salts, phosphonium salts and sulphonium salts. Preferred imidazolium salts have formula (I) 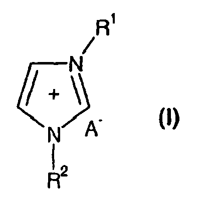 wherein
R¹ and R² are independently selected from the group consisting of a C₁-C₁₈ aliphatic group and a C₄-C₁₈ aromatic group; and
A⁻ is an anion.

Preferably, R¹ and R² are independently selected from the group consisting of methyl, ethyl, propyl and butyl.

Preferred ammonium salts have formula (II) 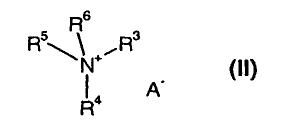 wherein
R³, R⁴, R⁵ and R⁶ are independently selected from the group consisting of a C₁-C₁₈ aliphatic group and a C₄-C₁₈ aromatic group; and
A' is an anion.

Preferably, R³, R⁴, R⁵ and R⁶ are independently selected from the group consisting of ethyl, propyl and butyl.

Preferred phosphonium salts have formula (III) 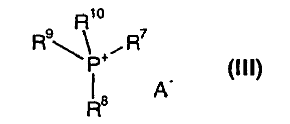 wherein
R⁷, R⁸, R⁹ and R¹⁰ are independently selected from the group consisting of a C₁-C₁₈ aliphatic group and a C₄-C₁₈ aromatic group; and
A⁻ is an anion.

Preferably, R⁷, R⁸, R⁹ and R¹⁰ are independently selected from the group consisting of ethyl and butyl.

Preferred pyridinium salts have formula (IV) 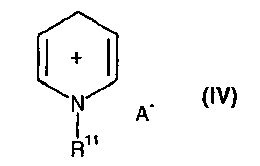 wherein
R¹¹ is selected from the group consisting of a C₁-C₁₈ aliphatic group and a C₄-C₁₈ aromatic group; and
A⁻ is an anion.

Preferably R¹¹ is ethyl or butyl.

Specific examples of ionic liquids include, but are not limited to, 1-butyl-3-methylimidazolium hexafluorophosphate, 1-hexyl-3-methylimidazolium hexafluorophosphate, 1-octyl-3-methylimidazolium hexafluorophosphate, 1-decyl-3-methylimidazolium hexafluorophosphate, 1-dodecyl-3-methylimidazolium hexafluorophosphate, 1-ethyl-3-methylimidazolium bis((trifluoromethyl)sulphonyl)-imidate, 1-hexyl-3-methylimidazolium bis((trifluoromethyl)sulphonyl)amide, 1-hexylpyridinium tetrafluoroborate, 1-octylpyridinium tetrafluoroborate, 1-butyl-3-methylimidazolium tetrafluoroborate, 1-methy-3-ethyl imidazolium chloride, 1-ethyl-3-butyl imidazolium chloride, 1-methy-3-butyl imidazolium chloride, 1-methy-3-butyl imidazolium bromide, 1-methy-3-propyl imidazolium chloride, 1-methy-3-hexyl imidazolium chloride, 1-methy-3-octyl imidazolium chloride, 1-methy-3-decyl imidazolium chloride, 1-methy-3-dodecyl imidazolium chloride, 1-methy-3-hexadecyl imidazolium chloride, 1-methy-3-octadecyl imidazolium chloride, 1-methy-3-octadecyl imidazolium chloride, ethyl pyridinium bromide, ethyl pyridinium chloride, ethylene pyridinium dibromide, ethylene pyridinium dichloride, butyl pyridinium chloride, and benzyl pyridinium bromide.

Preferred ionic liquids are 1-ethyl-3-methyl-imidazolium-trifluoroacetate, 1-butyl-3-methylimidazolium-trifluoroacetate, 1-ethyl-3-methyl-imidazolium-trifluoroacetate, 1-butyl-3-methylimidazolium-hexafluoro-phosphate, 1-octyl-3-methyl-imidazolium hexafluorophosphate, 1-hexyl-3-methy-imidazolium hexafluorophosphate, 1-butyl-3-methyl-imidazolium hexafluorophosphate, 1-butyl-3-methyl-imidazolium tetrafluoroborate, 1-ethyl-3-methylimidazolium-tetrafluoroborate, 1-octyl-3-methyl-imidazolium-bromid, 1-ethyl-3-methylimadazolium-trifluorosulfonate, 1-butyl-3-methyl-imidazolium-trifluorosulfonate,1-butyl-3-methyl-imidazolium trifluoromethanesulfonate, 1-ethyl-3-methyl-imidazolium trifluoromethanesulfonate, and 1-ethyl-3-methyl-imidazolium bis-(trifluoromethanesulfonyl)-imidate. Most preferably, the ionic liquid is selected from 1-ethyl-3-methyl-imidazolium-trifluorosulfonate, 1-octyl-3-methyl-imidazolium hexafluorophosphate and 1-hexyl-3-methylimidazolium hexafluorophosphate. A combination of ionic liquids may also be used.

In one embodiment of the invention, the microparticles are prepared by (i) dissolving or dispersing an active agent in a biocompatible, biodegradable polymer; (ii) dissolving the polymer containing the active agent in an ionic liquid; and (iii) removing the ionic liquid to form microparticles, e.g. by washing the microparticles.

In another embodiment of the invention, the microparticles are prepared by (i)' dissolving or dispersing an active agent in an ionic liquid; (ii)' dissolving the ionic liquid containing the active agent in a biocompatible, biodegradable polymer; and (iii)' removing the ionic liquid to form microparticles, e.g. by washing the microparticles.

In a further embodiment of the invention, the microparticles are prepared by (i)" dissolving or dispersing an active agent in a biocompatible, biodegradable polymer and an ionic liquid to form a mixture; (ii)" adding a solvent and at least one surfactant to the mixture; and (iii)" removing the ionic liquid to form microparticles, e.g. by washing the microparticles.

In a further embodiment of the invention, the microparticles are prepared by (i)'" dissolving or dispersing a biodegradable polymer in an ionic liquid; (ii)"' emulsification of the resulting solution in a lipophilic phase; (iii)"' adding a solution of an active agent to the emulsion to form microparticles and (iv)"' removing the ionic liquid e.g. by washing the microparticles.

Examples of surfactants include:
1) reaction products of a natural or hydrogenated castor oil and ethylene oxide. The natural or hydrogenated castor oil may be reacted with ethylene oxide in a molar ratio of from about 1:35 to about 1:60, with optional removal of the polyethyleneglycol component from the products. Various such surfactants are commercially available. The polyethyleneglycol-hydrogenated castor oils available under the trademark CREMOPHOR are especially suitable. Particularly suitable are CREMOPHOR RH 40, which has a saponification number of about 50 to 60, an acid number less than about 1, a water content (Fischer) less than about 2%, an n_{D}⁶⁰ of about 1.453 to 1.457 and an HLB of about 14 to 16; and CREMOPHOR RH 60, which has a saponification number of about 40 to 50, an acid number less than about 1, an iodine number of less than about 1, a water content (Fischer) of about 4.5 to 5.5%, an n_{D}²⁵ of about 1.453 to 1.457 and an HLB of about 15 to 17. An especially preferred product of this class is CREMOPHOR RH40. Also suitable are polyethyleneglycol castor oils such as that available under the trade name CREMOPHOR EL, which has a molecular weight (by steam osmometry) of about 1630, a saponification number of about 65 to 70, an acid number of about 2, an iodine number of about 28 to 32 and an n_{D}²⁵ of about 1.471.
   Similar or identical products which may also be used are available under the trademarks NIKKOL (e.g. NIKKOL HCO-40 and HCO-60), MAPEG (e.g. MAPEG CO-40h), INCROCAS (e.g. INCROCAS 40), and TAGAT (for example polyoxyethylene-glycerol-fatty acid esters e.g. TAGAT RH 40; and TAGAT TO, a polyoxyethylene-glycerol-trioleate having a HLB value of 11.3; TAGAT TO is preferred). These surfactants are further described in Fiedler, H. P. "Lexikon der Hilfsstoffe für Pharmazie, Kosmetik und angrenzende Gebiete", Editio Cantor, D-7960 Aulendorf, 4th revised and expanded edition (1996).
2) Polyoxyethylene-sorbitan-fatty acid esters, also called polysorbates, for example mono- and tri-lauryl, palmityl, stearyl and oleyl esters of the type known and commercially available under the trademark TWEEN (Fiedler, loc.cit., p.1300-1304) including the products TWEEN
   20 [polyoxyethylene(20)sorbitanmonolaurate],
   21 [polyoxyethylene(4)sorbitanmonolaurate],
   40 [polyoxyethylene(20)sorbitanmonopalmitate],
   60 (polyoxyethylene(20)sorbitanmonostearate],
   65 [polyoxyethylene(20)sorbitantristearate],
   80 [polyoxyethylene(20)sorbitanmonooleate],
   81 [polyoxyethylene(5)sorbitanmonooleate],
   85 [polyoxyethylene(20)sorbitantrioleate].

Especially preferred products of this class are TWEEN 40 and TWEEN 80.
3) Polyoxyethylene fatty acid esters, for example polyoxyethylene stearic acid esters of the type known and commercially available under the trademark MYRJ (Fiedler, loc. cit., p.834-835). An especially preferred product of this class is MYRJ 52 having a D²⁵ of about 1.1., a melting point of about 40 to 44°C, an HLB value of about 16.9., an acid value of about 0 to 1 and a saponification no. of about 25 to 35.
4) Polyoxyethylene-polyoxypropylene co-polymers and block co-polymers, for example of the type known and commercially available under the trademark PLURONIC, EMKALYX and POLOXAMER (Fiedler, loc. cit., p. 959). An especially preferred product of this class is PLURONIC F68, having a melting point of about 52°C and a molecular weight of about 6800 to 8975. A further preferred product of this class is POLOXAMER 188.
5) Dioctylsulfosuccinate or di-[2-ethylhexyl]-succinate (Fiedler, loc. cit. p. 107-108).
6) Phospholipids, in particular lecithins (Fiedler, loc. cit. p. 943-944). Suitable lecithins include, in particular, soybean lecithins.
7) Propylene glycol mono- and di-fatty acid esters such as propylene glycol dicaprylate (also known and commercially available under the trademark MIGLYOL 840), propylene glycol dilaurate, propylene glycol hydroxystearate, propylene glycol isostearate, propylene glycol laurate, propylene glycol ricinoleate, propylene glycol stearate and so forth (Fiedler, loc. cit. p. 808-809).
8) Polyoxyethylene alkyl ethers such as those commercially available under the trademark BRIJ, e.g., Brij 92V, Brij 93 and Brij 35.
9) Tocopherol esters, e.g., tocopheryl acetate and tocopheryl acid succinate.
10) Docusate salts, e.g., dioctylsulfosuccinate or related compounds, such as di-[2-ethylhexyl]-succinate (Fiedler, loc. cit., p. 107-108).

A combination of surfactants may also be used.

Examples of solvents include: alkyl acetates, e.g. linear or branched C₁₋₆ alkyl acetates, such as ethyl acetate, isopropyl acetate, and butyl acetate; lower alkyl alcohols, e.g. linear or branched C₁₋₆ alkyl alcohols such as methanol, ethanol, isopropanol, and butanol; aliphatic C₆₋₁₂ hydrocarbons, e.g. isooctane and n-heptane; aromatic hydrocarbons such as toluene, benzene; dialkyl ketones, e.g. acetone, methyl isobutyl ketone; acetonitrile; dialkyl ethers, e.g. diisopropyl ether and diethylene ether; polyvinyl alcohol. A combination of solvents may also be used.

Examples of lipophilic phases e.g. lipophilic components, include: liquid paraffins (Fiedler loc. cit., p. 1141), silicon oils e.g. dimethicone (Fiedler loc. cit., p. 465), mixtures of middle-chain triglycerides e.g. Miglyol 812® (Fiedler loc. cit., p. 1008) and oleic acid oleoyl esters e.g. Cetiol® (Fiedler loc. cit., p. 337). A combination of lipophilic components may be used.

Prior to administration to a patient, the microparticles may be suspended in an acceptable pharmaceutical liquid vehicle. The pharmaceutical liquid vehicle may optionally contain thickeners such as carboxymethyl cellulose, etc., surfactants, such as polyoxyethylene sorbitan monooleate, polysorbate 20; and polyoxyalkylene derivatives of propylene glycol, etc., and/or isotonicity agents, such as sugars, salts, etc.

The microparticles can be mixed by size or by type so as to provide for the delivery of active agent to a patient in a multiphasic manner and/or in a manner that provides different active agents to the patient at different times, or a mixture of active agents at the same time.

The microparticles may vary in size, ranging from submicron to millimeter diameters. Preferably, the average particle size of the microparticles is from 1-500 microns, more preferably 10-200 microns, e.g. about 20 to about 40, e.g. about 30 microns. The microparticles may show a rough surface.

The amount of active agent, for example, peptide, present in the microparticles depends on a number of factors. Such factors include, but are not limited to, the following: the desired daily release dosage, the loading capacity within the polymer, physical-chemical properties as water solubility, the desired time of delivery, and polymer properties, e.g., degradation time and molecular weight. The exact amount of active agent may be ascertained by loading experiments, *in vitro* and *in vivo* dissolution rates and pharmacokinetic trials. Preferably, the microparticles contain an active agent in an amount from 0.1-90 weight percent (wt %), based on the weight of the polymeric matrix, more preferably 2-75 wt %.

The release time of the active agent from the microparticle may be from one or two weeks to about 12 months.

The following non-limiting examples illustrate further aspects of the invention.

### Example 1

### A) Preparation of Bromocryptine mesylate Microparticles

Bromocryptine mesylate, 3 mg is dissolved and/or dispersed in poly(D,L-lactid) R202, 15 mg. This combination is dissolved in 1 ml of 1-ethyl-3-methyl-imidazolium-trifluorsulfonate and stored in a centrifuge tube. A lipophilic phase is prepared which contained paraffin and Brij 92V which is commercially available from Uniquema Everberg, as an emulsifier. The phases are emulsified with an IKA Vibrofix VF1 Electronic high shear homogenizer. To the resulting emulsion, small amounts of water is added step wise under further homogenization/emulsification. Polyvinyl alcohol was added to the emulsion. Water is added to the emulsion, and the emulsion is centrifuged yielding microparticles having a diameter of from 1 to 20 microns.

Example 1A is repeated except that 1-ethyl-3-methyl-imidazolium-trifluorsulfonate is replaced by the same amount of 1-octyl-3-methyl-imidazolium hexafluorophosphate or 1-hexyl-3-methyl-imidazolium hexafluorophosphate.

The process described in Example 1A may be up scaled e.g. 1000 fold.

### B) Preparation of Bromocryptine mesylate Microparticles

Bromocryptine mesylate, 3 mg is dissolved and/or dispersed in poly(D,L-lactid) R202, 15 mg. This combination is dissolved in 1 ml of 1-ethyl-3-methyl-imidazolium-trifluorsulfonate. A lipophilic phase is prepared which contained paraffin and Brij 93 which is commercially available from Uniquema Everberg, as an emulsifier. The phases are emulsified with an IKAMAG® RCT magnetic stirrer. To the resulting emulsion, small amounts of water are added step wise under further homogenization/emulsification. Polyvinyl alcohol was added to the water. The resulting suspension/emulsion is put in a centrifuge tube already containing 3 ml water and is centrifuged yielding microparticles having a diameter of from 25 to 40 microns.

Example 1B is repeated except that 1-ethyl-3-methyl-imidazolium-trifluorsulfonate is replaced by the same amount of 1-octyl-3-methyl-imidazolium hexafluorophosphate or 1-hexyl-3-methyl-imidazolium hexafluorophosphate.

The process described in Example 1B may be up scaled e.g. 1000 fold.

### Example 2

### Preparation of BSA Microparticles:

25 mg poly(D,L-lactid) R202 are dissolved in 0.5 ml of 1-ethyl-3-methyl-imidazolium-trifluorsulfonate. This solution is emulsified in 4.5 ml liquid paraffin using an IKAMAG® RCT magnetic stirrer. To stabilize the resulting emulsion 1 % Brij 93 was added to the liquid paraffin. An aqueous solution of 50 mg/ml bovine albumin fraction V is prepared. 200 µl of this solution are added step wise under further homogenization/emulsification. The resulting suspension/emulsion is put in a centrifuge tube already containing 5 ml water and is centrifuged yielding microparticles having a diameter of from 25 to 40 microns.

Example 2 is repeated except that 1-ethyl-3-methyl-imidazolium-trifluorsulfonate is replaced by the same amount of 1-octyl-3-methyl-imidazolium hexafluorophosphate or 1-hexyl-3-methyl-imidazolium hexafluorophosphate.

The process described in Example 2 may be up scaled e.g. 1000 fold.

### The encapsulation of BSA is shown by UV/Vis spectroscopy and gel-electrophoresis (native PAGE).

### Example 3

### Preparation of unloaded Microparticles:

Poly(D,L-lactid) R202, 15 mg is dissolved in 1 mi of 1-ethyl-3-methyl-imidazolium-trifluorsulfonate. A lipophilic phase is prepared which contained paraffin and Brij 93 which is commercially available from Uniquema Everberg, as an emulsifier. The phases are emulsified with an IKAMAG® RCT magnetic stirrer. To the resulting emulsion, small amounts of water are added step wise under further homogenization/emulsification.

Polyvinyl alcohol was added to the water. The resulting suspension/emulsion is put in a centrifuge tube already containing 3 ml water and is centrifuged yielding microparticles having a diameter of from 25 to 40 microns.

Example 3 is repeated except that 1-ethyl-3-methyl-imidazolium-trifluorsulfonate is replaced by the same amount of 1-octyl-3-methyl-imidazolium hexafluorophosphate or 1-hexyl-3-methyl-imidazolium hexafluorophosphate.

The process described in Example 3 may be up scaled e.g. 1000 fold.

Microparticles prepared according to the invention have the following advantages:
(i) an active agent or drug substance and a polymer which are used to prepare the microparticles may co-dissolve in the ionic liquid which may eliminate an emulsification or drug suspending step in a process to prepare the microparticles;
(ii) the distribution of an active agent or drug substance in a polymer matrix may be more homogeneous in the microparticles;
(iii) the microparticles are free from halogenated hydrocarbon residues;
(iv) the microparticles are prepared essentially free of volatile solvents since the ionic liquids have essentially no vapor pressure;
(v) the particle size, shape, and density of the microparticles may be controlled by the choice of ionic liquid;
(vi) an active agent and a polymer may be co-dissolved in the ionic liquid; and
(vii) a peptide or protein co-dissolved with a polymer in the ionic liquid may not undergo potentially devastating stress conditions during emulsification or suspension of a protein/water phase in a polymer/ionic liquid phase.

While the invention has been described with particular reference to certain embodiments thereof, it will be understood that changes and modifications may be made by those of ordinary skill within the scope and spirit of the following claims:

## Claims

1. Microparticles comprising at least one active agent embedded within a biocompatible, biodegradable polymeric matrix, wherein said microparticles are prepared with an ionic liquid.

2. The microparticles according to Claim 1 wherein the ionic liquid has essentially no vapor pressure.

3. The microparticles according to Claim 5 wherein the ionic liquid has a vapor pressure of less than about 1 mm/Hg at 25 °C.

4. The microparticles according to Claim 1 wherein the ionic liquid is selected from the group consisting of: an imidazolium salt, pyridium salt, ammonium salt, phosphonium salt and sulphonium salt.

5. The microparticles according to Claim 1 wherein the ionic liquid is selected from the group consisting of: 1-butyl-3-methylimidazolium hexafluorophosphate, 1-hexyl-3-methylimidazolium hexafluorophosphate, 1-octyl-3-methylimidazolium hexafluorophosphate, 1-decyl-3-methylimidazolium hexafluorophosphate, 1-dodecyl-3-methylimidazolium hexafluorophosphate, 1-ethyl-3-methyl-imidazolium-trifluorosulfonate, 1-butyl-3-methylimidazolium-trifluorosulfonate, 1-ethyl-3-methylimidazolium bis((trifluoromethyl)sulphonyl)-imidate, 1-hexyl-3-methylimidazolium bis((trifluoromethyl)sulphonyl)amide, 1-ethyl-3-methylimidazolium-trifluoroacetate, 1-butyl-3-methyl-imidazolium-trifluoroacetate, 1-ethyl-3-methylimidazolium-tetrafluoroborate, 1-hexylpyridinium tetrafluoroborate, 1-octylpyridinium tetrafluoroborate, 1-butyl-3-methylimidazolium tetrafluoroborate, 1-methy-3-ethyl imidazolium chloride, 1-ethyl-3-butyl imidazolium chloride, 1-methy-3-butyl imidazolium chloride, 1-methy-3-butyl imidazolium bromide, 1-octyl-3-methyl-imidazolium-bromide, 1-methy-3-propyl imidazolium chloride, 1-methy-3-hexyl imidazolium chloride, 1-methy-3-octyl imidazolium chloride, 1-methy-3-decyl imidazolium chloride, 1-methy-3-dodecyl imidazolium chloride, 1-methy-3-hexadecyl imidazolium chloride, 1-methy-3-octadecyl imidazolium chloride, 1-methy-3-octadecyl imidazolium chloride, ethyl pyridinium bromide, ethyl pyridinium chloride, ethylene pyridinium dibromide, ethylene pyridinium dichloride, butyl pyridinium chloride, benzyl pyridinium bromide, and mixtures thereof.

6. The microparticles according to Claim 1 wherein the polymer is a co-polymer of poly(glycolic acid) and poly(D,L-lactic acid).

7. The microparticles according to Claim 1 wherein the active agent is selected from the group consisting of a peptide, protein, hormone, analgesic, anti-migraine agent, anti-coagulant agent, narcotic antagonist, chelating agent, anti-anginal agent, chemotherapy agent, sedative, anti-neoplastic, prostaglandin and antidiuretic agent, cerebral stimulant, pain management agent, antalkaloid, cardiovascular drug and agent for treating rheumatic condition.

8. The microparticles according to Claim 7 wherein the peptide or protein is selected from the group consisting of insulin, calcitonin, calcitonin gene-regulating protein, parathyroid hormone, GLP-1, atrial natriuretic protein, colony-stimulating factor, GM-CSF, betaseron, erythropoietin, α-interferon, β-interferon, γ-interferon, human growth hormone, octreotide, somatropin, somatotropin, somastostatin, somatomedins, luteinizing hormone releasing hormone, tissue plasminogen activator, growth hormone releasing hormone, oxytocin, estradiol, growth hormones, leuprolide acetate, factor VIII, interleukin-2, interleukin-3, interleukin-6, interleukin-14, and analogues and antagonists thereof.

9. Microparticles comprising at least one active agent embedded within a biocompatible, biodegradable polymeric matrix, and at least one ionic liquid.

10. A method for preparing microparticles comprising (i) dissolving or dispersing an active agent in a biocompatible, biodegradable polymer; (ii) dissolving the polymer containing the active agent in an ionic liquid; and (iii) removing the ionic liquid to form microparticles.

11. A method for preparing microparticles comprising (i)' dissolving or dispersing an active agent in an ionic liquid; (ii)' dissolving the ionic liquid containing the active agent in a biocompatible, biodegradable polymer; and (iii)' removing the ionic liquid to form microparticles.

12. A method for preparing microparticles comprising (i)" dissolving or dispersing an active agent in a biocompatible, biodegradable polymer and an ionic liquid to form a mixture; (ii)" adding a solvent and at least one surfactant to the mixture; and (iii)" removing the ionic liquid to form microparticles.

13. A method for preparing microparticles comprising (i)'" dissolving or dispersing a biodegradable polymers in an ionic liquid; (ii)"' emulsification of the resulting solution in a lipophilic phase; (iii)"' adding a solution of an active agent to the emulsion to form microparticles, and (iv)'" removing the ionic liquid.

14. The method according to Claim 12 wherein the surfactant is selected from the group consisting of a reaction products of a natural or hydrogenated castor oil and ethylene oxide, polyoxyethylene-sorbitan-fatty acid esters, polyoxyethylene fatty acid esters, polyoxyethylene-polyoxypropylene co-polymers and block co-polymers, dioctylsulfosuccinate or di-[2-ethylhexyl]-succinate, phospholipids, propylene glycol mono- and di-fatty acid esters, polyoxyethylene alkyl ethers, tocopherol esters, and docusate salts and combinations thereof.

15. The method according to Claim 12 wherein the solvent is selected from the group consisting of an alkyl acetate, lower alkyl alcohol, aliphatic C₆₋₁₂ hydrocarbon, aromatic hydrocarbon, dialkyl ketone, dialkyl ether, and combinations thereof.

16. The method according to claim 13 wherein the lipophilic phase is selected from the group consisting of liquid paraffins, silicon oils, mixtures of middle-chain triglycerides, oleic acid oleoyl esters and combinations thereof.
